Europäisches Patentamt

**European Patent Office**

**Office européen des brevets**

(11) Veröffentlichungsnummer: **0 081 109**
**A2**

(12) # EUROPÄISCHE PATENTANMELDUNG

(21) Anmeldenummer: 82110611.9

(22) Anmeldetag: 18.11.82

(51) Int. Cl.³: **A 61 F 13/02**
**A 61 N 1/42**

(30) Priorität: 04.12.81 DE 8135397 U

(43) Veröffentlichungstag der Anmeldung:
15.06.83 Patentblatt 83/24

(84) Benannte Vertragsstaaten:
AT BE CH DE FR GB IT LI LU NL SE

(71) Anmelder: **Energy-Pak Ltd.**
**Oberlindenberg 191**
**CH-9427 Wolfhalden(CH)**

(72) Erfinder: **Latzke, Arno W.**
**Oberlindenberg 191**
**CH-9427 Wolfhalden(CH)**

(74) Vertreter: **Werner, Hans-Karsten, Dr. et al,**
**Deichmannhaus am Hauptbahnhof**
**D-5000 Köln 1(DE)**

(54) **Magnetisches Heilpflaster für die Behandlung grösserer Flächen.**

(57) Ein magnetisches Heilpflaster für die Behandlung größerer Flächen besteht aus kugelförmigen, tablettenförmigen, ovalen oder rechteckigen Dauermagneten mit einem Durchmesser von 2 bis 12 mm und einer Dicke von 1 bis 10 mm, wobei diese Magnete mit Feldstärken von 300 bis 1500 Gauss auf einem Träger mit hautverträglichen Klebern in Abständen von 2 bis 20 mm geometrisch nach Plus- und Minuspolen geordnet angebracht sind.

Fig 1.

EP 0 081 109 A2

- 1 -

Gegenstand der Erfindung ist ein magnetisches Heilpflaster für die Behandlung größerer Flächen. Magnetische Heilpflaster zur magnetischen Therapie von Rheuma, Gelenkschmerzen, Ischias, Hexenschuß und sonstigen Erkrankungen sind bekannt. Es wurde jetzt gefunden, daß auch Narben erfolgreich mit Magnetpflastern behandelt werden können und dabei überraschenderweise wesentlich besser verheilen und nicht mehr oder in wesentlich geringerem Maße ihr unschönes Aussehen behalten. Dies ist insbesondere bei Brandwunden der Fall.

Aus dem deutschen Gebrauchsmuster 79 19 808 sind magnetische Heilpflaster bekannt, die aus einem Ferritplättchen und einem selbstklebenden Heftpflaster bestehen. Diese Heilpflaster haben sich bereits für viele Zwecke gut bewährt. Bei der Behandlung größerer Flächen ist es jedoch umständlich und aufwendig, viele Pflaster auf engem Raum nebeneinander zu befestigen. Außerdem ist es nicht ohne weiteres möglich, die Magnetpole geometrisch geordnet anzubringen. Es bestand somit die Aufgabe, für diese Zwecke ein verbessertes magnetisches Heilpflaster zu entwickeln.

Es wurde nun gefunden, daß man größere Flächen besonders wirkungsvoll und einfach behandeln kann, wenn man kugelförmige, tablettenförmige, ovale oder rechteckige Dauermagnete mit einem Durchmesser von 2 bis 12 mm (vorzugsweise 4 bis 6 mm) und einer Dicke von 1 bis 10 mm (vorzugsweise ca. 2 mm) verwendet, wobei diese Magnete Feldstärken von 300 bis 1500, vorzugsweise 700 bis 1000 Gauss, aufweisen sollen. Diese Dauermagnete werden auf einem Träger mit hautverträglichen Klebern in Abständen von 2 bis 20 mm, vorzugsweise 8 bis 12 mm, geometrisch geordnet nach Plus- und Minuspolen angebracht. Je nach dem gewünschten Zweck können der Kleberseite nur die Pluspole oder nur die Minuspole zugewandt sein. Für gewisse Fälle ist es jedoch besonders wirkungsvoll, wenn abwechselnd geometrisch angeordnet positive und negative Pole angebracht werden. Auch streifenförmige Anordnung von Plus- oder Minuspolen nebeneinander kommen prinzipiell für gewisse Anwendungen infrage. Als geometrische Anordnung werden vorzugsweise gleichschenklige Dreiecke oder Quadrate gewählt, da diese Anordnung eine gleichmäßige und dichte Packung der Magnete ermöglichen.

Die Träger der erfindungsgemäßen magnetischen Heilpflaster für große Flächen bestehen aus hautverträglichem Material wie Kunststoffolien, Textilien oder Schaumstoff. Insbesondere bei Schaumstoff ist es möglich, die Magnete soweit im Schaumstoff einzubetten, daß sie nicht mehr oder kaum noch aus dem Schaumstoff herausragen und somit nahezu drucklos auf die Haut aufgebracht werden können.

Vergleichende Untersuchungen über die Hauttemperatur und ihre Veränderung bei der Einwirkung der erfindungsgemäßen Magnetpflaster und herkömmlichen Pflastern mit durchblutungsfördernden Inhaltsstoffen, beispielsweise auf Basis von Extractum et Fructus Capsici, haben ergeben, daß bei den erfindungsgemäßen Magnetpflastern keine meßbaren Temperatursteigerungen entstehen, während bei herkömmlichen Heilpflastern mit durchblutungsfördernden Pflanzenextrakten Temperatursteigerungen von 3°C unterhalb des Pflasters und 1 bis 2°C in einem Abstand bis zu 10 cm vom Pflaster feststellbar sind. Die bei der Anwendung der erfindungsgemäßen Heilpflaster verspürte Wärmesteigerung ist somit subjektiv und beruht nur auf einer Veränderung der Stoffwechsellage in den Zellen und in dem umliegenden Bindegewebe, während bei herkömmlichen Pflastern mit Wirkstoffen eine gewisse

künstliche Entzündung durch höhere Durchblutung der Haut erzeugt wird.

Da es sich somit um zwei völlig verschiedene Wirkprinzipien handelt, ist es prinzipiell möglich, diese miteinander zu kombinieren. Dazu wird in einer bevorzugten Ausführungsform der erfindungsgemäßen Magnetpflaster eine selbstklebende Schicht gewählt, die zusätzlich eine durchblutungsfördernde Substanz enthält. Als durchblutungsfördernde Substanzen kommen alle bereits bekannten und in der Therapie bewährten Substanzen infrage. Besonders bevorzugt sind durchblutungsfördernde Substanzen aus Senf, Paprica oder Fructus capsici. Ein Gemisch Extractum et fructus capsici, entsprechend etwa 0,05 bis 5 %, vorzugsweise 0,1 bis 3 %, hat sich bei herkömmlichen Heilpflastern bewährt und ist deshalb auch prinzipiell geeignet, in dieser Menge in die selbstklebende Schicht der erfindungsgemäßen Magnetpflaster eingebracht zu werden.

Da erfindungsgemäße Magnetpflaster mit dem Zusatz durchblutungsfördernde Substanzen im doppelten Sinne wirksam sind, ist auch eine raschere und intensivere Wirung die Folge.

In den nachstehenden Beispielen sind einige Ausführungsformen der erfindungsgemäßen Magnetpflaster näher erläutert. Es ist jedoch für den Fachmann selbstverständlich, daß auch andere Ausführungsformen mit anderen Größen und anderen Materialien zu entsprechenden Ergebnissen führen werden.

- 4 -

## Beispiel 1

Auf ein selbstklebendes und hautverträgliches Pflaster aus Kunststoffolie oder Textilien werden in Abständen von 10 mm tablettenförmige Ferritplättchen mit einem Durchmesser von 5 mm und einer Dicke von 2 mm in einem quadratischen Muster aufgebracht. Die Ferritplättchen sind so angeordnet, daß die dem Pflaster zugewandte Seite ausschließlich Südpole aufweist. Dieses Pflaster wird in rechteckige Stücke von 60 x 100 mm geschnitten und verpackt. Für die Verpackung kann in üblicher Weise ein Wachspapier auf die klebende Schicht aufgebracht werden, welches vor der Benutzung entfernt wird.

In entsprechender Weise wird ein anderes Pflaster hergestellt, bei dem ausschließlich die Nordpole dem selbstklebenden Pflaster zugewandt sind.

In analoger Weise wird ein Pflaster hergestellt, bei dem die Ferritkerne hexagonal angeordnet sind und abwechselnd Nord- oder Südpole dem selbstklebenden Pflaster zugewandt haben.

## Beispiel 2

Auf eine 2 mm dicke Schaumstoffbahn aus Polyurethanschaum wird ein hautverträglicher Kleber aufgebracht. Auf diese Schicht wird eine 3 mm dicke zweite Schaumstoffbahn aufgebracht, in die in quadratischem Abstand von 12 mm zylinderförmige Dauermagnete eingebracht sind, die 6 mm Durchmesser und 3 mm Dicke aufweisen und eine Feldstärke von 450 bis 500 Gauss aufweisen. Auf diese Schicht wird eine zweite Schicht Klebstoff aufgebracht, so daß sowohl die Magnetplättchen als auch der Schaumstoff selbstklebend werden. Das Ganze wird mit Wachspapier abgedeckt und in Stücke von

- 5 -

60 x 100 mm geschnitten und verpackt. Vor der Benutzung wird das Wachspapier abgezogen und das Paket mit den Magneten auf die Haut aufgeklebt.

Beispiel 3

Eine Schaumstoffbahn von 10 mm Stärke wird in Abständen von 15 mm hexagonal mit Löchern von 8 mm Durchmesser versehen. In diese Löcher werden kugelförmige Dauermagnete mit einer Feldstärke von 700 Gauss eingebracht. Das ganze Gebilde wird mit einer beidseitig klebenden Kunststoffolie abgedeckt und die noch offene klebende Seite mit Wachspapier abgedeckt. Hieraus werden quadratische Stücke von 100 x 100 mm ausgeschnitten und verpackt. Vor der Verwendung wird das Wachspapier abgezogen und das ganze Pflaster auf die Haut aufgeklebt.

Beispiel 4

Ein textiles Pflaster mit selbstklebender Schicht und einem Gehalt von 3 % Extractum et Fructus Capsici wird in Abständen von 10 mm mit abgerundeten tablettenförmigen Ferritplättchen mit dem Durchmesser 5 mm und der Dicke 2 mm bestückt, und zwar so, daß die Nordpole der selbstklebenden Seite des Pflasters zugewandt sind. Das Ganze wird mit Wachspapier abgedeckt und verpackt. Vor Benutzung wird das Wachspapier wieder abgezogen und das Pflaster auf die Haut aufgeklebt.

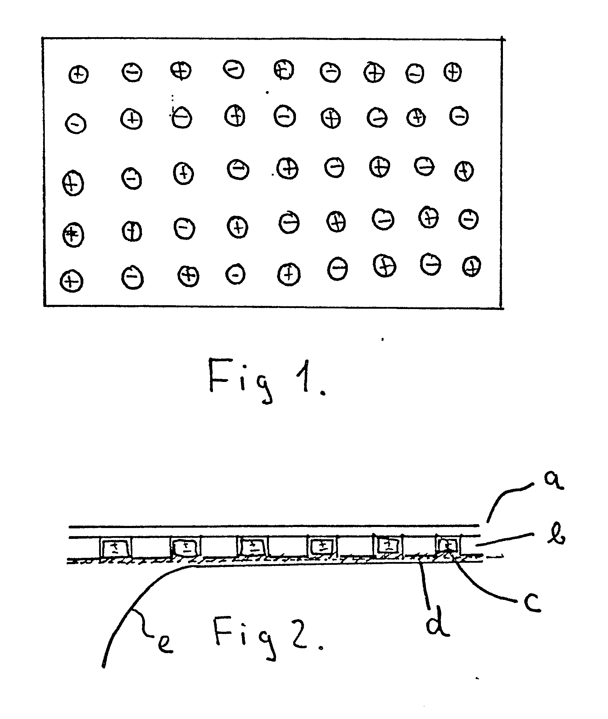

In der Figur 1 ist schematisch ein erfindungsgemäßes magnetisches Heilpflaster in der Aufsicht dargestellt, wobei die Magnete quadratisch angeordnet sind und abwechselnd plus und minus oben angeordnet ist.

Figur 2 zeigt den Schnitt durch ein Pflaster, welches aus einer Schaumstoffschicht a besteht, die selbstklebend beschichtet ist, auf die eine zweite durchlöcherte Schaumstoffschicht b aufgeklebt ist. In den Löchern befinden sich zylinderförmige Dauermagnete (c), die einheitlich den Minuspol nach außen aufweisen. Das ganze Pflaster ist mit einer hautverträglichen Kleberschicht (d) versehen, auf die eine Wachspapierschicht (e) aufgebracht ist, die vor der Benutzung abgezogen wird.

- 7 -

PATENTANSPRÜCHE

1. Magnetisches Heilpflaster für die Behandlung größerer Flächen, bestehend aus kugelförmigen, tablettenförmigen, ovalen oder rechteckigen Dauermagneten mit einem Durchmesser von 2 bis 12 mm und einer Dicke von 1 bis 10 mm, dadurch gekennzeichnet, daß die Magnete mit Feldstärken von 300 bis 1500, vorzugsweise 700 bis 1000 Gauss, auf einem Träger mit hautverträglichen Klebern in Abständen von 2 bis 20 mm, vorzugsweise 8 bis 12 mm, geometrisch nach Plus- und Minuspolen geordnet angebracht sind.

2. Magnetisches Heilpflaster gemäß Anspruch 1, dadurch gekennzeichnet, daß der Träger aus hautverträglichen Kunststoffolien, Textilien oder Schaumstoffen besteht.

3. Magnetisches Heilpflaster nach Anspruch 1 oder 2, dadurch gekennzeichnet, daß der hautverträgliche Kleber zusätzlich eine durchblutungsfördernde Substanz enthält.

4. Magnetisches Heilpflaster gemäß Anspruch 3, dadurch gekennzeichnet, daß die durchblutungsfördernde Substanz aus Senf, Paprica oder Fructus Capsici gewonnen wurde und in einer Menge von 0,05 bis 5 Gew.-% der selbstklebenden Schicht vorliegt.

0081109

Fig 1.

Fig 2.

a

b

c

d

e